# EUROPÄISCHE PATENTANMELDUNG

(11) **EP 1 661 515 A2**
(43) Veröffentlichungstag der Anmeldung: **31.05.2006**
(21) Anmeldenummer: 05090311.1
(22) Anmeldetag: 12.11.2005
(51) Int. Cl.: A61B 5/107, G01B 3/10

(54) **Vorrichtung zur elektronischen Erfassung von Massen, insbesondere von Körpermassen eines Patienten**

(30) Priorität: 13.11.2004 DE 202004017909 U
(71) Anmelder: Weihermüller & Voigtmann GmbH, 95448 Bayreuth (DE)
(72) Erfinder: Weihermüller, Stefan, 95444 Bayreuth (DE)
(74) Vertreter: Voigt, Günter

(57) **Zusammenfassung**

Es wird eine Vorrichtung zur Erfassung von Maßen, insbesondere von Körpermaßen eines Patienten oder von Oberflächenmaßen beschrieben, die mit Einrichtungen zur digitalen Abtastung des Maßbandes (10) und zur elektronischen Speicherung und Weiterleitung der Messwerte ausgestattet ist.

## Beschreibung

Die Erfindung bezieht sich auf eine Vorrichtung zur elektronischen Erfassung von Maßen, insbesondere von Körpermaßen eines Patienten.

Im medizinischen Bereich besteht in sehr vielen Fällen die Notwendigkeit, Körpermaße eines Patienten zu erfassen. Bis vor nicht allzu langer Zeit erfolgte dies mit Hilfe eines flexiblen, in seiner Länge praktisch nicht dehnbaren üblichen Maßbandes. Dabei musste die Vielzahl der zu ermittelnden Werte von Hand notiert werden. Verständlicherweise ist ein solches Vorgehen recht umständlich, da nach jeder Messung das Maßband zur Seite gelegt und der Meßwert auf einem Datenblatt festgehalten werden muss. Gleichzeitig eröffnet sich in diesem Zusammenhang verständlicherweise eine Vielzahl von Fehlerquellen.

In der Medizintechnik sind auch bereits verschiedene kontaktfreie Meßverfahren mit Scannern bekannt geworden, die aber aus verschiedenen Gründen nicht uneingeschränkt einsetzbar sind.

Teilweise werden die Maße eines Arms oder Beins eines Patienten auch mit Hilfe eines bedruckten Arm- oder Bein-Strumpfes und einer Digital-Kamera ermittelt.

Lichtstreifenmessungen mit Hilfe der Photogrammetrie kommen ebenfalls zur Anwendung.

Im Handwerk zum Einsatz kommende Digitalmessbänder aus Metall sind zur Anwendung im medizinischen Bereich oder in Bereichen mit sensitiven Oberflächen nicht geeignet.

Aus der NL-A 1019003 ist ein elektronisches Rollmaß bekannt, das speziell für die Vermessung menschlicher Körperteile bestimmt ist, um den gemessenen Werten entsprechend Hilfsmittel wie Strümpfe, Korsetts oder dergleichen individuell anfertigen zu können. Das Maßband ist dabei auf eine Trommel aufgewickelt, deren jeweilige Winkel-Position schlupffrei auf ein Messrad übertragen wird. Ein Problem ergibt sich daraus, dass die Winkelmessung in eine Längenmessung umgesetzt und dabei die je nach Wickelzustand der Trommel unterschiedlichen Umfänge berücksichtigt werden müssen. Die sich ergebenden Abweichungen erlauben keine direkte Zuordnung von Winkelstellung bzw. Umdrehungszahl der Trommel zur abgewickelten Maßband-Länge. Die sich bei dieser Messeinrichtung ergebenden Meßfehler werden durch ein entsprechendes Programm softwaremäßig korrigiert.

Aus der deutschen Offenlegungsschrift DE 44 26 009 A 1 ist eine elektronische Längenmesseinheit mit berührungslosen Inkrement-Abtastsystem bekannt, bei der das die Inkremente tragende Band nicht wie üblich parallel, planliegend oder fest auf der Gesamtlänge der longitudinalen Messachse montiert wird, sondern auf einer Aufrolltrommel auf- und abgewickelt wird, und wobei während des Messvorgangs das das Inkrement tragende Band am Längenmess-Abtastkopf vorbeigleitet, der die inkrementalen Logitudinal-Messsignale berührungslos abtastet. Auf der konkaven Innenseite des Messbandes sind dabei mit Plus- und Minus-Polen magnetisierte Schichten aufgebracht, die als Zählimpulse (Inkremente) berührungslos induktiv mit dem Abtastknopf aufgenommen werden. Bei dieser bekannten Längenmesseinheit muss zu den inkrementalen Messwerten jeweils ein Ausgangswert hinzugefügt werden.

Es ist weiter eine Einrichtung zur Erfassung von Maßen, insbesondere von Körpermaßen eines Patienten, bekannt, bei der ein Bandmaß verwendet wird, das auf eine Trommel aufgewickelt ist, die sich in einem Aufnahmebehälter mit Rückholfeder befindet. Diese Einrichtung verfügt auch über eine Vorrichtung zur digitalen Abtastung der Längenmarkierung des Bandmaßes.

Der vorliegenden Erfindung liegt die Aufgabe zu Grunde, eine solche Vorrichtung dahingehend zu verbessern, dass bei einer möglichst einfachen Meßdaten-Erfassung eine automatische Auslesung und Weiterleitung der jeweiligen Messwerte erfolgen kann.

Die Lösung dieser Aufgabe erfolgt mit Hilfe der kennzeichnenden Merkmale des Anspruchs 1.

Vorteilhafte Weiterbildungen ergeben sich aus den Unteransprüchen.

Die Erfindung wird nachfolgend unter Bezugnahme auf die Zeichnungsfiguren beispielsweise beschrieben. Es zeigen:
Fig. 1a und 1b eine schematische Darstellung der erfindungsgemäßen Messeinrichtungen,

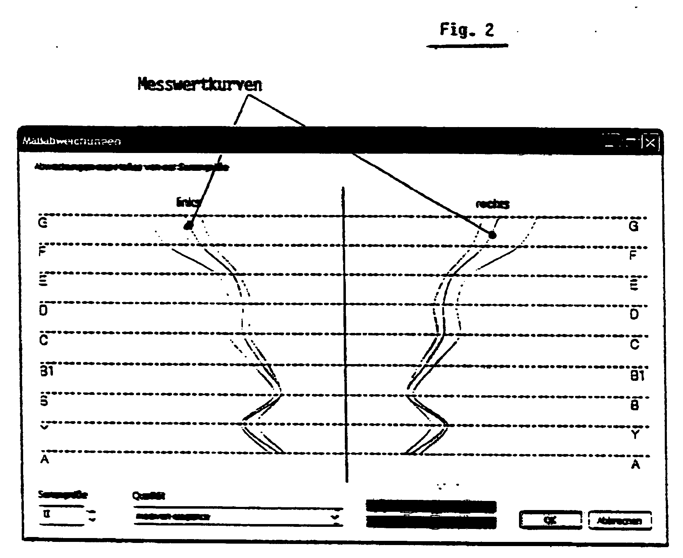

Fig. 2 eine als Kurvenverlauf dargestellte Messwert-Reihe,
Fig. 3 eine schematische Darstellung einer erfindungsgemäßen Meßkulisse,
Fig. 4 ein Maßband mit einer Mess-Schablone für das elektronische Erfassen der Messwerte und
Fig. 5 einen Aufnahmebehälter für das Bandmaß mit einer Einrichtung zur elektronischen Erfassung der Bandspannung beim Messvorgang.

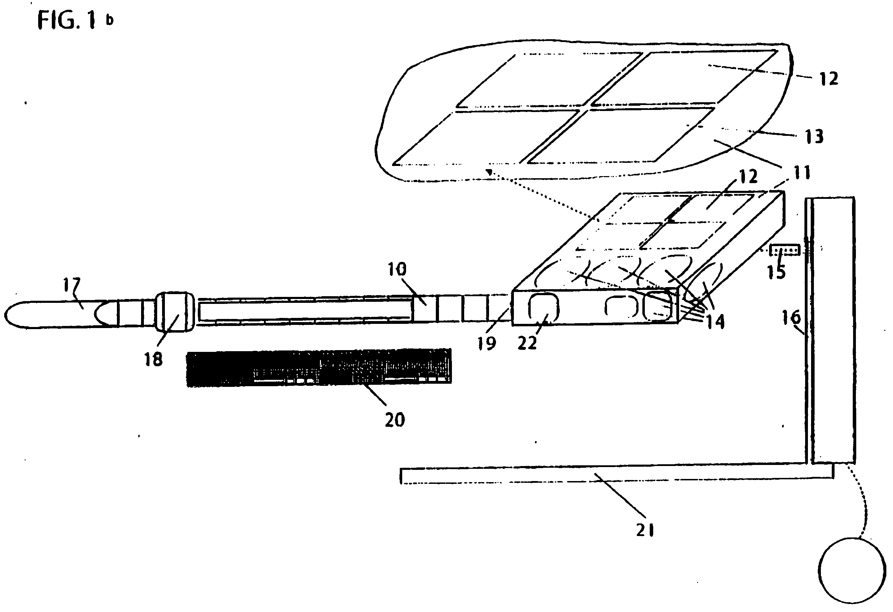

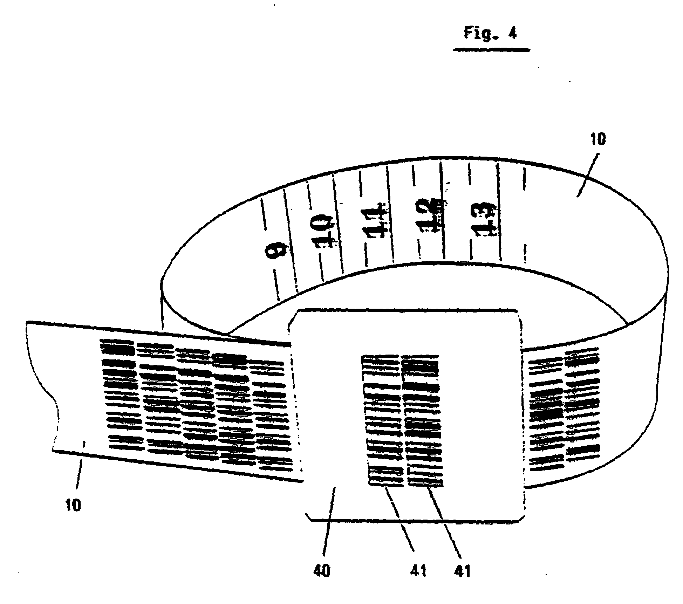

In Fig. 1a ist ein in Längsrichtung praktisch nicht dehnbares, aber flexibles - grundsätzlich bereits bekanntes - Roll-Maßband 10 dargestellt, das auf seiner einen Seite mit der bekannten und üblichen Zentimenter-Teilung und auf der anderen Seite mit unmittelbar aufeinander folgenden Barcodes versehen ist, die für die zu erfassenden Messwerte stehen. Einzelheiten des Roll-Bandmaßes 10 ergeben sich aus Fig. 4.

Die Folge der die Maße wiedergebenden einzelnen Barcodes erfolgt je nach Genauigkeitsanforderungen in einem Abstand von wenigen Millimetern, vorzugsweise in einem Abstand von 5 bis 10 Millimetern.

Ein Massband mit einer Zentimenter-Einteilung und ggfs. auch mit einer Millimeter-Einteilung ist den einschlägigen Verkehrskreisen bereits allgemein bekannt und den einschlägigen Kreisen vertraut. Mit Hilfe der auf der zweiten Seite des vorliegenden Maßbandes vorhandenen Barcode-Teilung besteht im vorliegenden Fall jedoch auch die Möglichkeit einer elektronischen Erfassung der jeweiligen Messwerte durch Scannen. Da die gescanten Werte anschließend als elektronische Daten vorliegen, können sie unmittelbar elektronisch erfasst, gespeichert, weiterverarbeitet und weitergeleitet werden.

Die Messwert-Erfassung erfolgt dabei in an sich bekannter Weise mittels eines Scanners. Das Massband kann auch durch eine entsprechende Führung 40 mit mindestens einem Fenster 41 zum Auslesen der entsprechenden Barcodes des Massbandes hindurchgeführt werden. Der jeweils im Fenster der Führung 40 sichtbare Barcode des Bandmaßes 10 entspricht dabei dem gemessenen Umfang.

Für den Messvorgang wird das eine Ende des Bandmaßes 10 mit der Führung 40 fest verbunden und das andere - freie - Ende des Bandmaßes 10 in einer Führung durch die erwähnte Führung 40 hindurchgezogen.

Werden zwei unmittelbar benachbarte Fenster mit etwas weniger als der halben Höhe das Barcodes in der Führung 40 vorgesehen, so können die Barcodes beider benachbarter Fenster ausgelesen und aus diesen beiden Werten ein Mittelwert gebildet werden. Durch diese Massnahme wird - wie später noch im einzelnen erläutert wird - die Messgenauigkeit verdoppelt, ohne dass ein Barcode mit anderer Teilung verwendet werden müsste.

Die eigentliche Messwerterfassung erfolgt mit Hilfe üblicher Barcode-Scanner, die entweder über Kabel oder per Bluethooth mit einem Computer zur Erfassung der Messwerte verbunden sind.

Dabei können die über verschiedene Höhen 35 am Körper erfassten Werte (z.B. Umfangswerte) in systematischer Reihenfolge erfasst und gespeichert sowie ggfs. auch gemäss Fig. 1c als Kurvenverlauf 33 dargestellt werden und die sich so ergebenden Kurvenverläufe 33 mit als Hintergrund einblendbaren Normgrößen der Strümpfe oder anderer Bekleidungsstücke verglichen werden. Verlaufen die erfassten Messwerte innerhalb der vorgegebenen Toleranzgrenzen, so kann auf Normgrößen zurückgegriffen werden. Anderenfalls muss eine Einzelanfertigung nach den erfassten Maßen erfolgen.

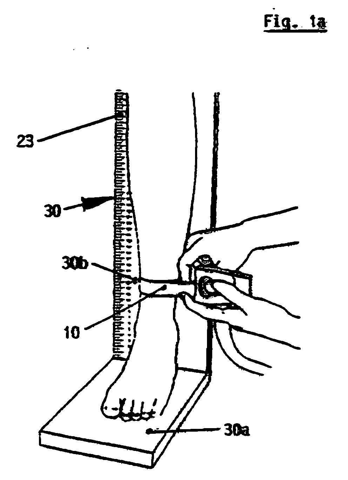

Das Bandmaß 10 kann auch gemäss Fig. 1b in einem Aufnahmebehälter 11 in grundsätzlich bekannter Art und Weise auf eine Trommel mit Rückholfeder aufgewickelt sein. Auf der Oberfläche des Aufnahmebehälters 11 können Displays 12, 13 für die Messwertanzeige bzw. Messpunktvorgabe sowie Bedienelemente 14 für bestimmte Funktionen wie Rückholen des Massbandes, Löschen der Messwerte, Bedienung des Up/Down-Cursors, Setup etc. angeordnet sein.

Die jeweils zugeordnete Lage bzw. Höhe der Messung kann über eine Barcode-Markierung auf einer starren Mess-Kulisse 30 oder auf einer entsprechenden Matte abgetastet werden. Die Kulisse 30 beteht aus einem Fussteil 30a und einem rechtwinklig dazu verlaufenden Beinteil 30b besteht. Der Fuß des Patienten wird auf das Fußteil 30a der Mess-Kulisse 30 so aufgesetzt, dass sich das Bein vor dem Bein-Teil 30b der Mess-Kulisse befindet. Bei Umfangs-Messungen in bestimmten Höhen des Beins werden der Umfang vom Massband sowie die zugehörige Höhe 23 von der Mess-Kulisse 30 gescannt.

Bei Verwendung eines Roll-Bandmaßes in einem Aufnahmebehälter 11 kann dieser ggfs.auch über einen Aufnahmezapfen 15 oder in anderer geeigneter Weise an einer Messtange 16 stufenlos höhenverstellbar angeordnet werden. Die jeweiligen Höhen-Positionen der erwähnten Mess-Stange 16 können dabei ebenfalls über Barcodes digital erfasst und auf einem der zuvor erwähnten Displays 12, 13 angezeigt werden.

Dadurch wird es möglich, zwei Maße unterschiedlicher Art (zwei Längenmessungen oder auch Umfangsmessung und Längen- bzw- Höhenmessung) gleichzeitig parallel - sozusagen in einer Messfolge - zu ermitteln.

Das Ende des Bandmaßes 10 gemäss Fig. 1b ist mit einem Zug- bzw. Haltegriff 17 sowie mit einem optischen Messkopf 18 versehen, der in geeigneter Weise, beispielsweise über eine aufladbare Energiezelle oder über drahtlos empfangene Erregungssignale, die notwendige Ernergie erhält.

Ein Messkopf 22 weist eine Abtasteinrichtung mit einem Auslesefenster auf, das etwas weniger als die Häfte der Höhe des Barcodes in Längsrichtung des Massbandes aufweist und mit dessen Hilfe die Barcodes auf dem Massband ausgelesen werden. Die im Auslesefenster erfassten Werte entsprechen den jeweiligen Messlängen, da die Barcodes im Bereich des Auslesefensters ein Massstab für die jeweils gemessenene Werte darstellen und somit die Länge des ausgezogenen Bandmaßes 10 wiedergeben. Je nach spezieller Aufgabenstellung können die die Messwerte darstellenden Barcodes auf dem Massband im Abstand von 1 bis 10 mm - bei Bedarf auch in anderen Abständen - wechseln. Bei der Vermessung von Körpermassen ist es im allgemeinen ausreichend, etwa alle 5 mm den Barcode und damit die Längenangabe zu ändern.

Es kann vorteilhaft sein, zwei Auslese-Fenster 41 mit jeweils etwas weniger als der halben Höhe der Barcodes in Längsrichtung des Massbandes unmittelbar nebeneinander anzuordnen und aus den so erfassten Messwerten einen Mittelwert zu bilden. Liegen beide Auslese-Fenster 41 am gleichen Barcode, so entspricht der ermittelte Wert exakt dem Wert des Barcodes. Liegen die beiden Auslesefenster jedoch an unterschiedlichen Barcodes an, so wird über den Rechner der Mittelwert der beiden erfassten Barcodes gebildet. Auf diese Art und Weise kann die Messgenauigkeit bei einer 5-mm-Folge der Barcodes auf 2,5 mm gesteigert werden.

Werden beispielsweise Umfänge gemessen, kann das Bandmaß 10 um den zu messenden Umfang gelegt und der Messkopf 18 nach Umfassung des zu messenden Umfangs dort auf das Bandmaß 10 aufgelegt werden. Der Messkopf 18 erfasst den Barcode auf dem Bandmaß 10 und ermittelt daraus den Meßwert und den zu messenden Umfang.

Das Bandmaß 10 kann neben dem Barcode ggfs. auch zusätzlich mit einer Farbcodierung 20 versehen sein. Vorteilhafterweise sind die beiden Oberflächen des Bandmaßes 10 mit unterschiedlichen Codierungen, d.h. mit einer Codierung einerseits und einer üblichen Zentimeter-Einteilung oder einer Skaleninformation andererseits versehen.

Das Bandmaß 10 kann bei Bedarf, insbesondere bei etwaigem Verschleiss, problemlos ausgetauscht werden.

Der Aufnahmezapfen 15 des Aufnahmebehälters 11, der in die Scharniere der Meßstange 16 eingeschoben werden kann, enthält einen dritten Messkopf zur Feststellung der Länge/Höhe entlang der Meßstange 16. Für die Messwerterfassung gilt hier das zuvor Dargelegte ganz entsprechend.

In Abhängigkeit vom voreinzustellenden Maßprogramm und der Feststellung, ob Aufnahmebehälter 11 und Meßstange 16 Kontakt haben, wird zusätzlich der parallele Längen- bzw. Höhenwert ermittelt.

Der Aufnahmezapfen 15 der Meßstange 16 kann auch mit einer Winkelverstellung versehen sein. Dabei kann auch die Winkelstellung digital erfasst und können die so erfassten Messwerte digital weitergegeben und beispielsweise auf einem Diplay 12, 13 angezeigt werden.

Aufnahmebehälter 11 und Meßstange 16 können ggfs. auch mit einer Rastvorrichtung versehen sein.

Als eine denkbare Ergänzung bzw. Substitution für die Meßstange 16 kann für die parallele Messung einer Strecke/Höhe auch eine opto-elektronische Zusatzeinrichtung im Aufnahmebehälter 11 vorgesehen werden. Dadurch wird es möglich, eine zweite Strecke mit optischer Fixierung des Zielpunktes und anschließender Messung zu erfassen und gleichzeitig den entsprechende Winkel zu bestimmen.

Über Batterien, die beispielsweise neben Speicherchips und Sender in der Meßstange 16 angeordnet sind, werden die notwendigen Energien für die Messprogramme und die Datenanzeige zur Verfügung gestellt.

Die Erfassung der jeweiligen Messwerte erfolgt nach Betätigung entsprechender Auslöseelemente, beispielsweise Tasten am Aufnahmebehälter 11, mit einem mit dem Fußteil verbundenen oder einzelnen Fußschalter oder in geeigneter Weise am Meßkopf 18.

Die Logik ist so ausgelegt, dass mit dem Maßband 10 mehrere Messungen bzw. Meßabläufe nacheinander ausgeführt und in der jeweils zutreffenden Reihenfolge und unter Hinzugabe weiterer Codierungen auf den Displays angezeigt bzw. in einem Speicher abgelegt werden können. Mit einer Basisstation können mehrere Datensammlungen parallel oder auch mehrere Maßbänder gleichzeitig verwaltet werden. Dazu gehört auch der Upload von notwendigen Daten wie Messobjekt-Codierung zum Wiedererkennen bei späterer Suche oder spezifischen Maßblatt-Logiken auf das jeweilige Bandmaß 10.

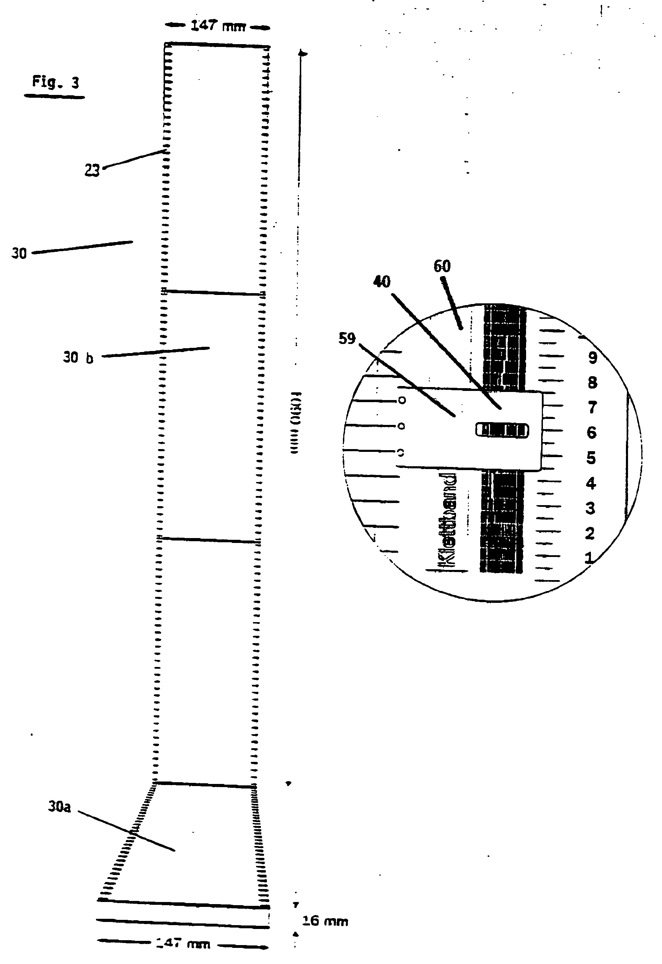

Die in Fig. 3 mit ihrer Codierung 23 beispielsweise dargestellte Mess-Kulisse 30 ist mit mehreren Codierungs-Spuren (Barcodes) versehen, die den mm-Bereich, den cm-Bereich, den dm-Bereich und den m-Bereich wiedergeben.

Parallel zu diesen Codierungs-Spuren kann auch ein Klettband 60 angeordnet werden. Ist das Ablese-Fenster 40 mit einem entsprechenden Gegenstück 59 versehen, so kann das Ablese-Fenster 40 für das Scannen des Barcodes vorübergehend am Klettband 60 fixiert werden kann.

Anstelle der Mess-Kulisse 30 kann selbstverständlich auch eine entsprechende Mess-Matte zur Anwendung kommen, die auf einer Liege oder einem Bett ausgelegt werden kann und die grundsätzlich die gleiche Funktion wie die Mess-Kulisse 30 hat.

In Fig. 4 ist ein Bandmaß 10 dargestellt, das weitgehende Übereinstimmung mit einem üblichen Roll-Bandmaß aufweist, aber nur auf einer Seite eine übliche mm- oder cm-Teilung aufweist, auf der anderen Seite dagegen eine Folge von Barcodes. Ein solches Bandmaß 10 wird mit seinem einen Ende an einer Führung 40 befestigt. Das andere Ende des Bandmaßes 10 wird durch die Führung 40 derart hindurchgezogen, dass die Barcode-Folge mittels eines Scanners erfasst werden kann - dies kann ggfs. auch in dem Auslese-Fenster 41 bzw. in den Auslese-Fenstern 41 geschehen. Die in dem bzw. den Fenstern 41 erscheinenden Barcodes geben die zu messende Länge wieder und können mit einem Scanner ausgelesen, anschließend gespeichert und weitegeleitet werden. Dies wurde bereits weiter oben geschildert.

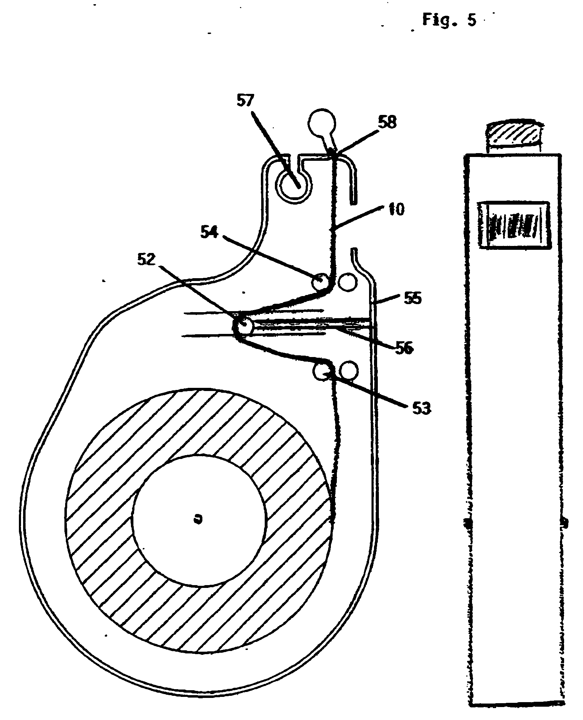

In Fig. 5 ist ein Aufnahmebehälter 11 mit einer integrierten Rückholfeder für das Bandmaß dargestellt. Das Bandmaß 10 wird um den zu erfassenden Körperteil gelegt und das freie Ende des Bandmaßes 10 in einer Halterung 57 am Aufnahmebehälter 11 in unmittelbarer Nähe des Eintrittspunktes 58 des Bandmaßes 10 im Aufnahmebehälter 11 fixiert.

Die Messwerte sind nur dann miteinander vergleichbar und damit verwertbar, wenn Sie bei einer in einem Toleranzbereich liegenden Bandspannung gemessen werden. Unter starker Bandspannung zieht sich der Körperteil verständlicherweise in einem gewissen Umfang zusammen und die Messwerte werden geringer.

Zum Erhalt verwertbarer Messergebnisse ist daher auch die Kenntnis der Bandspannung während des Messvorgangs erforderlich. Es gibt zwar die Möglichkeit, die Bandspannung über die Rückholfeder im Aufnahmebehälter weitgehend konstant zu halten. Infolge äußerer Einflüsse oder durch altersbedingte Veränderungen ist eine präzise Einhaltung einer konstanten Bandspannung allein mit der Rückholfeder jedoch nicht gewährleistet.

Es empfiehlt sich daher, auch die Bandspannung zu erfassen und den einzelnen Messwerten zuzuordnen. Dies geschieht, indem das Bandmaß 10 innerhalb des Aufnahmebehälters 11 über Umlenkungelemente 52, 53, 54 geführt wird. Die Bandspannung versucht das Umlenkelement 52 in Richtung auf die Außenwand 55 des Aufnahmebehälters 11 zu drücken. Der dabei im Stützteil 56 auftretende Druck kann mit drucksensitiven elektronischen Elementen erfasst und gemeinsam mit den übrigen Messwerten gspeichert und/oder weitergeleitet werden.

Damit ergibt sich zu jedem Wert einer Umfangs- oder Längenmessung auch ein dokumentierbarer Wert der Bandspannung während des Messvorgangs, wodurch der Aussagewert der Umfangs- oder Längenmesswerte ganz erheblich erhöht wird.

Bei einer Längenmessung wird der Endpunkt des Bandmaßes 10 nicht in der entsprechenden Halterung 57 des Aufnahmebehälters 11 fixiert, sondern am Ende des in seiner Länge zu messenden Teils.

Die erhaltenen elektronischen Messwerte können in an sich bekannter Weise elektronisch gespeichert oder auch weitergeleitet werden.

## Patentansprüche

1. Einrichtung zur Erfassung von Maßen, insbesondere von Körpermaßen eines Patienten oder Oberflächenmaßen auf sensitiven Materialien, mit einem Bandmaß, einer Trommel für die Aufnahme des Bandmaßes und einem das Bandmaß mit Trommel umgebenden Aufnahmebehälter mit Rückholfeder sowie mit einer Abtasteinrichtung zur digitalen Abtastung des Maßbandes, **dadurch gekennzeichnet, dass** die Abtasteinrichtung ein Scanner oder ein am Ende des Maßbandes vorhandener und nach Umfassung eines zu messenden Körperteils auf das Maßband (10) aufzulegender oder mit der Messeinrichtung zu verbindender Messkopf (18) ist.

2. Einrichtung nach Anspruch 1, **dadurch gekennzeichnet, dass** das Bandmaß durch eine Führung mit einem Sichtfenster führbar ist.

3. Einrichtung nach Anspruch 1 oder 2, **dadurch gekennzeichnet, dass** das Massband zumindest einseitig mit einem Barcode versehen ist.

4. Einrichtung nach einem der Ansprüche 1 bis 3, **dadurch gekennzeichnet, dass** ein Speicher zur Erfassung der abgelesenen Werte vorhanden ist.

5. Vorrichtung nach einem der Ansprüche 1 bis 4, **dadurch gekennzeichnet, dass** eine Einrichtung zu Weiterleitung der erfassten Daten vorhanden ist.

6. Einrichtung nach einem der Ansprüche 1 bis 5, **dadurch gekennzeichnet, dass** eine Vorrichtung zur zusätzlichen Erfassung der Bandspannung beim Messvorgang vorhanden ist.

7. Einrichtung nach einem der Ansprüche 1 bis 6, **dadurch gekennzeichnet, dass** die Werte der Bandspannung über eine die Bandspannung abtastende und elektronische Messwerte liefernde Vorrichtung erfassbar und weiterleitbar sind.

8. Einrichtung nach einem der Ansprüche 1 bis 7, **dadurch gekennzeichnet, dass** mehrere gleichzeitig und parallel arbeitende Abtasteinrichtungen vorhanden sind.

9. Einrichtung nach einem der Ansprüche 1 bis 8 , **dadurch gekennzeichnet, dass** der Aufnahmebehälter (11) Displays (12, 13) für die Messwertanzeige aufweist.

10. Einrichtung nach einem der Ansprüche 1 bis 9, **dadurch gekennzeichnet, dass** der Aufnahmebehälter (1) Bedienelemente (14) für bestimmte Funktionen aufweist.

11. Einrichtung nach einem der Ansprüche 1 bis 10, **dadurch gekennzeichnet, dass** zusätzlich eine höhenverstellbare Messstange (16) zur Aufnahme des Aufnahmebehälters (11) vorhanden ist.

12. Einrichtung nach Anspruch 11, **dadurch gekennzeichnet, dass** die Messtange (16) mit einem Aufnahmezapfen (15) für den Aufnahmebehälter (11) versehen ist.

13. Einrichtung nach einem der Ansprüche 1 bis 12, **dadurch gekennzeichnet, dass** das Ende des Maßbandes (10) einen Zug- und Haltegriff aufweist.
